# EUROPEAN PATENT APPLICATION

(11) **EP 2 942 060 A1**
(43) Date of publication of application: **11.11.2015**
(21) Application number: 15156144.6
(22) Date of filing: 29.09.2010
(51) Int. Cl.: A61K 38/16, A61K 38/17, A61K 31/7105, A61K 31/7088, A61P 3/04, A61P 3/10, A61K 38/04

(54) **Use of Protein Kinase C Delta (PKCD) Inhibitors to Treat Diabetes, Obesity and, Hepatic Steatosis**

(30) Priority: 29.09.2009 US 246821 P
(62) Divisional of application: 10821157.4
(71) Applicant: Joslin Diabetes Center, Inc., Boston, MA 02215 (US)
(72) Inventor: Kahn, Ronald C., West Newton, MA Massachusetts 02465 (US); Bezy, Olivier, Milton, MA Massachusetts 02186 (US)
(74) Representative: Williams, Gareth Owen

(57) **Abstract**

Described are methods of improving insulin sensitivity, and treating fatty liver disease (including non-alcoholic steatohepatitis (NASH)), by administering specific inhibitors of PKC delta.

## Description

### CLAIM OF PRIORITY

This application claims the benefit of U.S. Provisional Patent Application Serial No. 61/246,821, filed on September 29, 2009, the entire contents of which are hereby incorporated by reference.

### FEDERALLY SPONSORED RESEARCH OR DEVELOPMENT

This invention was made with Government support under Grant No. DK31036 and DK33210 awarded by the National Institutes of Health. The Government has certain rights in the invention.

### TECHNICAL FIELD

This invention relates to methods for the use of specific inhibitors of protein kinase C (PKC) delta isoform (PKCd) to treat diabetes, obesity, hepatic steatosis and related insulin resistant syndromes.

### BACKGROUND

Obesity and Type II Diabetes (T2D) have become major health concerns and are projected to increase even more worldwide within the next decades.

### SUMMARY

The present inventors have demonstrated, using multiple approaches, that PKCd is a major regulator of the insulin resistance and is at least one of the genetic modifiers of the diabetic risk between these two strains of mice. The importance of PKCd has been established by findings of differences in expression levels, and a showing that PKCd can regulate whole body and hepatic insulin sensitivity by liver-specific overexpression, whole body knock-out and by liver-specific reduction in PKCd. PKCd also appears to be a major modulator of the risk of development of hepatic steatosis in mice on high fat diet.

Thus the methods described herein include administration of a specific inhibitor of PKCd to a mammal to improve insulin sensitivity and treat or prevent (i.e., reduce risk of) the metabolic complications of insulin resistance, including fatty liver disease and diabetes/glucose intolerance.

In one aspect, the invention describes the use of a specific inhibitors of Protein Kinase C delta isoform (PKCd) as described herein for improving insulin sensitivity in a mammal, and/or for treating or preventing fatty liver disease (FLD) in a mammal.

In one aspect the invention provides methods for improving insulin sensitivity in a mammal. The methods include selecting a mammal on the basis that they are in need of improved insulin sensitivity; and administering to the mammal a therapeutically effective dose of one or more specific inhibitors of Protein Kinase C delta isoform (PKCd). In some embodiments, the methods further include evaluating insulin sensitivity in the subject, before, during, and/or after administration of the inhibitor.

In another aspect, the present invention provides methods for treating or preventing fatty liver disease (FLD) in a mammal. The methods include selecting a mammal on the basis that they have or are at risk of developing FLD, and administering to the mammal a therapeutically effective dose of one or more specific inhibitors of Protein Kinase C delta isoform (PKCd). In some embodiments, the methods further include evaluating fatty liver disease in the subject, before, during, or after administration of the inhibitor.

In some embodiments of the methods described herein, the mammal is obese or has visceral adiposity. In some embodiments, the mammal has type 2 diabetes. In some embodiments, the mammal has Nonalcoholic Steatohepatitis (NASH) or is at risk of developing NASH.

In some embodiments of the methods described herein, the inhibitor is a small molecule or peptide or peptidomimetic, e.g., selected from the group consisting of KAI-980, rottlerin, bisindolylmaleimide I, bisindolylmaleimide II, bisindolylmaleimide III, bisindolylmaleimide IV, calphostin C, chelerythrine chloride, ellagic acid, Go 7874, Go 6983, H-7, Iso-H-7, hypericin, K-252a, K-252b, K-252c, melittin, NGIC-I, phloretin, staurosporine, polymyxin B sulfate, protein kinase C inhibitor peptide 19-31, protein kinase C inhibitor peptide 19-36, protein kinase C inhibitor (EGF-R Fragment 651-658, myristoylated), Ro-31-8220, Ro-32-0432, safingol, sangivamycin, and D-erythro-sphingosinc. In some embodiments, the inhibitor is another inhibitor described herein.

In some embodiments of the methods described herein, the inhibitor is a PKC delta-specific inhibitory nucleic acid, e.g., a small interfering RNA or antisense that binds specifically to and reduces levels of PKC delta.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

In the following, All results are expressed as means ± SEM. Significance was established using the Student's t test and analysis of variance when appropriate. Differences were considered significant at p < 0.05.
FIG. 1A is a bar graph showing levels of PKCd mRNA in C57BL6/J mice on regular chow diet (CD) and high fat diet (HFD) (left panel, n=8 per group, *: p<0.003);
FIG. 1B shows the results of Western blot analysis of PKCd protein expression in liver of 24 weeks old CD vs. 18 weeks HFD treated C57BL6/J and 129SvEv mice.
FIG. 1C is a bar graph showing levels of PKCd mRNA in liver of 6 weeks old C57BL6/J and 129SvEv mice (left panel, n=5 per group, *: p<0.001)
FIG. 1D is a bar graph showing levels of PKCd mRNA in liver of newborn C57BL6/J and 129SvEv mice (n=7 per group, *: p<0.004).
FIG. 1E shows the results of Western blot analysis of PKCd protein expression in liver of newborn C57BL6/J and 129SvEv mice.
FIG. 1F is a schematic illustration of *Prkcd, Tkt, Cphx* and *Chdh* genes localization on mouse chromosome 14.
FIG. 1G is a bar graph showing copy number variation for *Prkcd, Tkt* anf *Cphx* genes between C57BL/6J and 129SvEv mice lines (n=6 per groups). Results were normalized to *Chdh* (*: p<0.00003). Genomic DNA was extracted using DNeasy blood & tissue kit from QIAGEN (Valencia, CA).
FIGs. 2A-D are bar graphs showing levels of PKCd mRNA in liver as measured by qPCR for Ob/Ob and Ob/+ mice (n=9-15 per group, *: p<0.004) (2A); IRlox vs. LIRKO mice (right panel, n=7-8 per group, *: p<0.04) (2B); Leptin and/or insulin treated ob/ob mice (n=6 per group) (2C); and LPS-treated C57BL/6J and 129SvEv mice (n=5 per group) (2D).
FIG. 2E shows a Western blot analyzing protein expression in liver of tunicamycin-treated animals for PKCd, Bip and actin expression. Each lane represents an individual animal.
FIGs. 2F and 2G show the results of qPCR analysis of mRNA expression of PKCd in control, streptozotocin and insulin rescued mice (n=13/11/14 per group) (2F); and control, streptozotocin and phlorizin-rescued mice (n=13/11/14 per group) (2G). Samples were treated as described above.
FIG. 3A is a bar graph showing body weight of wild type vs. PKCd KO mice (n=8 per group, *: p<0.02).
FIG. 3B is a line graph showing glucose tolerance test of wild type vs. PKCd KO mice (n=5 per group, *: p<0.03).
FIGs. 3C-3D are bar graphs showing glucose infusion rate (GIR) (3C) and hepatic glucose production (HPG) (3D) during euglycemic-hyperinsulinemic clamps of WT vs. PKCd KO mice (n=4 per group, *: p<0.05).
FIG. 3E is a photomicrograph showing hematoxylin and eosin stained histological sections of liver from wild type and PKCd KO mice (200X magnification).
FIG. 3F is a bar graph showing the results of qPCR analysis of mRNA for gluconeogenic and lipogenic genes in liver at the end of the euglycemic-hyperinsulinemic clamp in control vs. PKCd KO mice (n=4 per group, *: p<0.04).
FIG. 3G is an image showing Western blot analysis of proteins in the insulin signaling pathway in liver at the end of the euglycemic-hyperinsulinemic clamp. Each lane represents an individual animal.
FIG. 4A is a line graph showing the results of glucose tolerance tests (GTT) in mice overexpressing GFP (white squares) or PKCd (black dots) in the liver (n=13 per group).
FIG. 4B is a line graph showing the results of pyruvate tolerance test (PTT) in mice overexpressing GFP (white square) or PKCd (black dots) in the liver (n=9 per group).
FIG. 4C is a line graph showing the results of insulin tolerance test (ITT) in mice overexpressing GFP (white square) or PKCd (black dots) in the liver (n=19 per group).
FIG. 4D is a line graph showing the results of serum insulin and cholesterol levels in mice overexpressing GFP or PKCd in the liver.
FIG. 4E is a pair of photomicrographs of liver sections stained with eosin from mice overexpressing GFP (top panel) or PKCd (bottom panel) in the liver (200X magnification).
FIG. 4F is a pair of bar graphs showing triglycerides levels in liver (left graph) and blood (right graph) of 2 hours fasted mice overexpressing GFP or PKCd in the liver (n=8 per group, *p<0.01).
FIG. 5A is an image showing the results of Western blot analysis of insulin signaling pathway in liver of mice overexpressing GFP or PKCd in the liver, 5 minutes after intraperitoneal injection of insulin or vehicle as described in Experimental Procedures. Each lane represents an individual animal.
FIG. 5B is a bar graph showing the results of qPCR analysis of lipogenic gene expression in liver of mice overexpressing GFP or PKCd (n=6 per group, *: p<0.01 ).
FIG. 5C is a bar graph showing the results of qPCR analysis of gluconeogenic genes in liver of mice overexpressing GFP or PKCd (n=6 per group, *: p<0.05).
FIG. 5D is an image showing the results of Western blot analysis of PKCd and SREBP1c expression in livers from mice overexpressing GFP or PKCd in the liver.
FIG. 5E is an image showing the results of Western blot analysis of Foxo1 and lamin A/C expression in nuclear extracts of livers from mice overexpressing GFP or PKCd in the liver. In western blots each lane represents an individual animal.
FIG. 6A shows the validation of effective DNA recombination by PCR analysis of genomic DNA (left panel), qPCR measurement of PKCd mRNA expression (middle panel) and Western blot analysis of PKCd protein (right panel) in livers of PKCd -floxed mice after administration of empty or Cre recombinase expressing adenovirus.
FIG. 6B is a bar graph showing the results of glucose tolerance test of PKCd-floxed mice injected with empty (white squares) or Cre recombinase expressing adenovirus (black dotes) following 10 weeks of CD (dashed lines) or HFD (plain lines) (n=6 per group).
FIG. 6C is an image showing the results of Western blot analysis of insulin signaling pathway in liver of PKCd-floxed mice injected with empty or Cre recombinase expressing adenovirus following 10 weeks of CD or HFD and 5 minutes after injection of insulin or vehicle. Each lane represents an individual animal.
FIG. 6D is a schematic illustration of the targeting strategy used to generate PKCd conditional knock-out mice model.

### DETAILED DESCRIPTION

The importance of genetic background in defining the susceptibility to develop obesity and metabolic syndrome is of great importance in the development of new, effective therapies. The is apparent in both humans and mice. Two mouse lines, C57BL/6J ("B6 mice") and 129SvEv ("129 mice"), were previously identified as having different metabolic responses to aging and high fat diet. Indeed, over an 18-week period, B6 mice gained more weight, had higher levels of insulin and leptin, and showed greater glucose intolerance than 129 mice on a regular chow diet, and their phenotypic differences were even more exacerbated with high fat feeding. Genome-wide scans identified several quantitative trait loci, including a quantitative trait locus that was linked with hyperinsulinemia/insulin resistance on chromosome 14. One of the genes in this locus was Prkcd, encoding the novel protein kinase C delta (PKCd).

Several members of the family of protein kinase C (PKCs) have been implicated in the past in the regulation of insulin resistance and metabolism. Despite PKCd being one of the most extensively studied, it has only recently been implicated in the regulation of the insulin signaling pathway in in-vitro studies (see, e.g., Talior et al., Am. J. Physiol. Endocrinol. Metab. 288(2):E405-11 (2005); and Talior et al., Am. J. Physiol. Endocrinol. Metab. 285(2):E295-302 (2003)). Notably, several studies demonstrated the ability of PKCd to inhibit IRS1 by serine phosphorylation (see, e.g., Waraich et al., J. Biol. Chem. 283(17):11226-33 (2008)). However, the possible role of PKCd in the regulation of in-vivo insulin sensitivity still remained to be established.

The results described herein demonstrate that PKCd expression is higher in liver of obesity-prone B6 mice than in obesity resistant 129, as well as increased in liver of several insulin resistance mice models. PKCd null mice were more glucose tolerant than WT littermates. During euglycemic-hyperinsulinemic clamps, mice globally invalidated for the Prkcd gene displayed increased insulin sensitivity, including enhanced suppression of hepatic gluconeogenesis. In addition, the livers of PKCd null mice had improved insulin signaling and decreased gene expression of gluconeogenic and lipogenic pathways. In contrast, liver-specific overexpression of PKCd induced hepatic insulin resistance, glucose and pyruvate intolerances and led to the onset of hyperglycemia, hyperinsulinemia, and hepatosteatosis linked to insulin failure to repress gluconeogenesis and lipogenesis.

In order to assess the metabolic consequences of liver-specific ablation of PKCd in diet-induced T2D mice, Prkcd loxP mouse model was created. Repression of hepatic PKCd improved glucose tolerance, restored hepatic insulin signaling and decreased hepatosteatosis in high fat fed mice. The study of insulin signaling pathway activation in liver of all three mice models established a direct correlation between PKCd levels, p70S6K activation and IRS1 Ser307 phosphorylation. These results define PKCd as a major regulator of hepatic insulin sensitivity and demonstrate its implication in the onset of the metabolic syndrome during development of obesity.

### Methods of Treatment

The methods described herein include methods for the treatment or prevention of disorders associated with impaired insulin sensitivity or fatty liver disease (FLD), including hepatic steatosis and type 2 diabetes. In some embodiments, the FLD is non-alcoholic steatohepatitis (NASH). Generally, the methods include administering a therapeutically effective amount of a compositing comprising a specific inhibitor of PKCd as an active agent as described herein, to a subject who is in need of, or who has been determined to be in need of, such treatment.

As used in this context, to "treat" means to ameliorate or reduce the risk of at least one symptom of the disorder associated with impaired insulin sensitivity, or FLD. For example, a treatment administered to a subject with impaired insulin sensitivity can result in an improvement in insulin sensitivity, and a return or approach to normoglycemia. To "prevent" means to reduce risk of disease; a prevention need not reduce risk by 100%. Administration of a therapeutically effective amount of a compound described herein for the treatment of a condition associated with FLD will result in decreased or no increase in intra-cytoplasmic accumulation of triglyceride (neutral fats), and an improvement or no decline in liver function.

### Impaired Insulin Sensitivity

In some embodiments, the methods include selecting a subject on the basis that they have impaired insulin sensitivity, then administering a dose of an inhibitor of PKCd as described herein. Determining whether a subject has impaired insulin sensitivity can include reviewing their medical history, or ordering or performing such tests as are necessary (e.g., weighing the subject or calculating body fat percentage) to establish a diagnosis.

As used herein, the term "insulin resistant" or "impaired insulin sensitivity" refers to a subject in which one or more of the body's normal physiological responses to insulin are impaired or lost. Insulin resistance in a subject is defined as a reduced biological effect of endogenous or exogenous insulin. Insulin resistance is associated with a number of conditions in humans, including increased risk of developing type 2 diabetes. Insulin resistance is the pivotal feature of metabolic syndrome, which is a cluster of abnormalities that create risk for many of our most common medial conditions, including glucose intolerance, dyslipidemias, non-alcoholic fatty liver, cardiovascular disease, Alzheimer's disease and even some cancer. A diagnosis of insulin resistance in a subject can be made, and monitored, using any method known in the art, including the oral glucose tolerance test (OGTT), IV glucose tolerance test (FSIVGTT), insulin tolerance test (ITT), insulin sensitivity test (IST), and continuous infusion of glucose with model assessment (CIGMA), or the glucose clamp. See, e.g., Krentz, Insulin Resistance (Wiley-Blackwell, 2002); de Paula Martins et al., Eur. J. Obst. Gynecol. Reprod.Biol.,133 (2):203-207.

### Obesity, Body Mass Index (BMI) and Visceral Adiposity

In some embodiments, the present methods include selecting a subject on the basis that they are obese, then administering a dose of an inhibitor of PKCd as described herein. Determining whether a subject is obese can include reviewing their medical history, or ordering or performing such tests as are necessary (e.g., weighing the subject or calculating body fat percentage) to establish a diagnosis. In some embodiments, the methods include selecting subject on the basis that they are obese based on a determination of body mass index (BMI) in the subject. BMI is determined by weight relative to height, and equals a person's weight in kilograms divided by height in meters squared. (BMI=kg/m²). Accepted interpretations are given in Table 1.

**Table 1**

| **Category** | **BMI** |
|---|---|
| Underweight | ≤ 18.5 |
| Normal weight | 18.5-24.9 |
| Overweight | 25-29.9 |
| Obese | ≥ 30 |

Thus the methods described herein can include determining a subject's height (e.g., by measuring the subject or reviewing the subject's medical history), determining a subject's weight (e.g., by weighing the subject or reviewing the subject's medical history), and calculating BMI from the values determined thereby. Alternatively, the methods described herein can include reviewing a subject's medical history to determine their BMI (e.g., where the BMI has already been calculated).

In addition to generalized obesity, it is known that individual with central obesity (also called visceral obesity or visceral adiposity) are at particularly high risk for development of FLD and metabolic syndrome (see below). Visceral obesity is assessed clinically as increased waist-hip ratio or increased weight circumference. It may also be assessed more precisely by imaging methods including DEXA scanning and MRI.

### The Metabolic Syndrome

In some embodiments, the methods include determining whether a subject has the metabolic syndrome, and selecting the subject if they do have the metabolic syndrome, then administering a dose of an inhibitor of PKCd as described herein. Determining whether a subject has the metabolic syndrome can include reviewing their medical history, or ordering or performing such tests as are necessary to establish a diagnosis.

The metabolic syndrome, initially termed Syndrome X ((Reaven, Diabetes 37(12):1595-1607 (1988)), refers to a clustering of obesity, dyslipidemia, hypertension, and insulin resistance. All components of the metabolic syndrome are traditional risk factors for vascular disease. As used herein, the metabolic syndrome is defined by the presence of at least 3 of the following: abdominal obesity (excessive fat tissue in and around the abdomen, as measured by waist circumference: e.g., greater than 40 inches for men, and greater than 35 inches for women), fasting blood triglycerides (e.g., greater than or equal to 150 mg/dL), low blood HDL (e.g., less than 40 mg/dL for men, and less than 50 mg/dL for women), high blood pressure (e.g., greater than or equal to 130/85 mmHg) and/or elevated fasting glucose (e.g., greater than or equal to 110 mg/dL). In some embodiments, levels of these criteria may be higher or lower, depending on the subject; for example, in subjects of Asian ancestry; see, e.g., Meigs, "Definitions and Mechanisms of the Metabolic Syndrome." Current Opinions in Endocrinology and Diabetes, 13(2):103-110 (2006). A determination of the presence of metabolic syndrome can be made, e.g., by reviewing the subject's medical history, or by reviewing test results.

Based on data from the Third National Health and Nutrition Examination Survey (NHANES III) approximately 24% of the adults in the United States qualify as having the metabolic syndrome (Ford et al., JAMA 287(3):356-359 (2002)). Insulin resistance is now felt to be central in the pathogenesis of these related disorders. Recently, a central role for inflammation has been postulated in this cluster of diseases.

### Fatty Liver Disease (FLD)

In some embodiments, the methods include determining whether a subject has FLD, and selecting the subject if they do have FLD, then administering a dose of an inhibitor of PKCd as described herein. Determining whether a subject has FLD can include reviewing their medical history, or ordering or performing such tests as are necessary to establish a diagnosis. FLD can be caused by excessive alcohol consumption (alcoholic hepatitis), drugs (such as valproic acid and corticosteroids (e.g., cortisone or prednisone)), excessive Vitamin A, and obesity.

Most individuals with FLD are asymptomatic; the condition is usually discovered incidentally as a result of abnormal liver function tests or hepatomegaly, e.g., noted in an unrelated medical condition. Elevated liver biochemistry is found in 50% of patients with simple steatosis (see, e.g., Sleisenger, Sleisenger and Fordtran's Gastrointestinal and Liver Disease. Philadelphia: W.B. Saunders Company (2006)). In general, the diagnosis begins with the presence of elevations in liver tests that are included in routine blood test panels, such as alanine aminotransferase (ALT) or aspartate aminotransferase (AST). Even modest, subclinical increases in hepatic fat accumulation have been shown to be an early component in the progressive pathogenesis of metabolic syndrome (see, e.g., Almeda-Valdés et al., Ann. Hepatol. 8 Suppl 1:518-24 (2009); Polyzos et al., Curr Mol Med. 9(3):299-314 (2009); Byrne et al., Clin. Sci. (Lond). 116(7):539-64 (2009)).

Imaging studies are often obtained during evaluation process. Ultrasonography reveals a "bright" liver with increased echogenicity. Thus, medical imaging can aid in diagnosis of fatty liver; fatty livers have lower density than spleen on computed tomography (CT) and fat appears bright in T1-weighted magnetic resonance images (MRIs). Making a differential diagnosis of Nonalcoholic Steatohepatitis (NASH), as opposed to simple fatty liver, is done using a liver biopsy. For a liver biopsy, a needle is inserted through the skin to remove a small piece of the liver. NASH is diagnosed when examination of the tissue with a microscope shows fat along with inflammation and damage to liver cells. If the tissue shows fat without inflammation and damage, simple fatty liver or Nonalcoholic Fatty Liver Disease (NAFLD) is diagnosed. Thus, histological diagnosis by liver biopsy is sought when assessment of severity is indicated.

### PKC-delta (PKCd)

The PKCd protein is a member of the Protein Kinase C family. In humans and in, this kinase has been shown to be involved in B cell signaling and in the regulation of growth, apoptosis, and differentiation of a variety of cell types. Alternatively spliced transcript variants encoding the same protein have been observed. PKCd has recently been identified as a therapeutic target for several indications, see, e.g., Yonezawa et al., Recent Pat DNA Gene Seq. 3(2):96-101 (2009); Shen, Curr Drug Targets Cardiovasc Haematol Disord. 3(4):301-7 (2003);

Exemplary PKCd sequences in humans include NM_006254.3 (nucleic acid, for variant 1, the longer variant; both variants encode the same protein); NP_006245.2 (protein); NM_212539.1 (nucleic acid, for variant 1, the shorter variant, which lacks an exon in the 5' UTR as compared to variant 1); and NP_997704.1 (protein). Human genomic sequence can be found at NC_000003.11 (Genome Reference Consortium Human Build 37 (GRCh37), Primary Assembly). PKCd is also known as MAY1; dPKC; MGC49908; nPKC-delta; and PRKCD.

### Small Molecule Inhibitors ofPKCd

A number of small molecule inhibitors of PKCd are known in the art. Examples include bisindolylmaleimide I, bisindolylmaleimide II, bisindolylmaleimide III, bisindolylmaleimide IV, calphostin C, chelerythrine chloride, ellagic Acid, Go 7874, Go 6983, H-7, Iso-H-7, hypericin, K-252a, K-252b, K-252c, melittin, NGIC-I, phloretin, staurosporine, polymyxin B sulfate, protein kinase C inhibitor peptide 19-31, protein kinase C inhibitor peptide 19-36, protein kinase C inhibitor (EGF-R Fragment 651-658, myristoylated), Ro-31-8220, Ro-32-0432, rottlerin, safingol, sangivamycin, and D-erythro-sphingosine. See, e.g., US2008/0153903. Other small molecule inhibitors of PKC are described in U.S. Pat. Nos. 5,141,957, 5,204,370, 5,216,014, 5,270,310, 5,292,737, 5,344,841, 5,360,818, 5,432,198, 5,380,746, and 5,489,608, (European Patent 0,434,057), all of which are hereby incorporated by reference in their entirety. These molecules belong to the following classes: N,N'-Bis-(sulfonamido)-2- amino-4-iminonaphthalen-1-ones; N,N'-Bis-(amido)-2-amino-4-iminonaphthalen- 1-ones; vicinal- substituted carbocyclics; 1,3-dioxane derivatives; 1,4-Bis-(amino-hydroxyalkylamino)- anthraquinones; furo-coumarinsulfonamides; Bis-(hydroxyalkylamino)-anthraquinones; and N- aminoalkyl amides, 2-[1-(3-Aminopropyl)-1H-indol-3-yl]-3-(1H-indol-3-yl)maleimide, 2-[[1-[2-(1-Methylpyrrolidino)ethyl]-1H-indol-3-yl]-3-(1H-indol-3-yl)maleimide, Go 7874. Other known small molecule inhibitors of PKC are described in the following publications (Fabre, S., et al. 1993. Bioorg. Med. Chem. 1, 193, Toullec, D., et al. 1991. J. Biol. Chem. 266, 15771, Gschwendt, M., et al. 1996. FEBS Lett. 392, 77, Merritt, J.E., et al. 1997. Cell Signal 9, 53., Birchall, A.M., et al. 1994. J. Pharmacol. Exp. Ther. 268, 922. Wilkinson, S.E., et al. 1993. Biochem. J. 294, 335., Davis, P.D., et al. 1992. J. Med. Chem. 35, 994), and belong to the following classes: 2,3-bis(1H-Indol-3-yl)maleimide (Bisindolylmaleimide IV); 2-[1-(3-Dimethylaminopropyl)-5-methoxyindol-3-yl]-3-(1H-indol-3-yl) maleimide (Go 6983); 2-{8-[(Dimethylamino)methyl]-6,7,8,9-tetrahydropyrido[1,2-a]indol-3-yl}-3-(1-methyl-1H-indol-3- yl)maleimide (Ro-32-0432); 2-[8-(Aminomethyl)-6,7,8,9-tetrahydropyrido[1,2-a]indol-3-yl]-3-(1-methyl-1H-indol-3-yl)maleimide (Ro-31-8425); and 3-[1-[3-(Amidinothio)propyl-1H-indol- 3-yl]-3-(1-methyl-1H-indol-3-yl)maleimide Bisindolylmalcimide IX, Methanesulfonate (Ro-31-8220) all of which are also hereby incorporated by reference in their entirety.

In some embodiments, the PKC-delta inhibitor is a peptide inhibitor or peptidomimetic thereof, e.g., comprising 4 to 25 residues of the first variable region of PKCd. In some embodiments, the PKC-delta inhibitor is KAI-9803 (KAI Pharmaceuticals, Inc., South San Francisco, Calif.); described in WO2009/029678, and other inhibitors listed therein, e.g., in Table 1 thereof. In some embodiments, the PKC-delta inhibitor is KID 1-1, amino acids 8-17 [SFNSYELGSL]) conjugated reversibly to the carrier peptide Tat (amino acids 43-58 [YGRKKKRRQRRR]) by disulfide bond as described in [9,11] (KAI Pharmaceuticals). Other peptide inhibitors are known in the art, e.g., as described in U.S. Patent No. 6,855,693, U.S. Patent Application Publication Nos. 2004/204364, 2003/211109, 2005/0215483, and 2006/0153867; WO2006017578; and U.S. Provisional Patent Application Nos. 60/881,419 and 60/945,285.

In some embodiments, the PKC-delta selective inhibitor is Rottlerin (mallatoxin) or a functional derivative thereof. The structure of Rottlerin is shown in FIG. 9 of US2009/0220503. In some embodiments, the PKC-delta selective inhibitor is Balanol or a Balanol analog (i.e., perhydroazepine-substitution analogs). Balanol is a natural product of the fungus Verticillium balanoides (Kulanthaivel et al., J Am Chem Soc 115: 6452-6453 (1993)), and has also been synthesized chemically (Nicolaou et al., J. Am Chem Soc 116: 8402-8403 (1994)). The chemical structure of balanol is shown in FIG. 10 of US 2009/0220503. Balanol and perhydroazepine-substitution analogs are disclosed in US 2009/0220503 (see, e.g., Table 2 therein). Other derivatives based upon the structure of mallatoxin or balanol can be made, wherein the core structure is substituted by C₁-C₆ groups such as alkyl, aryl, alkenyl, alkoxy, heteroatoms such as S, N, O, and halogens.

Additional PKCd-specific inhibitors are described in Int'l Pat. Appl. Nos. WO2004078118, WO2009029678, and U.S. Pat. Nos. 6,828,327, 6,723,830, 6,686,373 and 5,843,935.

All of the foregoing are incorporated by reference herein.

### Inhibitory Nucleic Acids Specific for PKCd

The methods described herein can include the administration of inhibitory nucleic acid molecules that are targeted to a PKCd RNA, e.g., antisense, siRNA, ribozymes, and aptamers.

### siRNA Molecules

RNAi is a process whereby double-stranded RNA (dsRNA, also referred to herein as si RNAs or ds siRNAs, for double-stranded small interfering RNAs,) induces the sequence-specific degradation of homologous mRNA in animals and plant cells (Hutvagner and Zamore, Curr. Opin. Genet. Dev.:12, 225-232 (2002); Sharp, Genes Dev., 15:485-490 (2001)). In mammalian cells, RNAi can be triggered by 21-nucleotide (nt) duplexes of small interfering RNA (siRNA) (Chiu et al., Mol. Cell. 10:549-561 (2002); Elbashir et al., Nature 411:494-498 (2001)), or by micro-RNAs (miRNA), functional small-hairpin RNA (shRNA), or other dsRNAs which are expressed *in vivo* using DNA templates with RNA polymerase III promoters (Zeng et al., Mol. Cell 9:1327-1333 (2002); Paddison et al., Genes Dev. 16:948-958 (2002); Lee et al., Nature Biotechnol. 20:500-505 (2002); Paul et al., Nature Biotechnol. 20:505-508 (2002); Tuschl, T., Nature Biotechnol. 20:440-448 (2002); Yu et al., Proc. Natl. Acad. Sci. USA 99(9):6047-6052 (2002); McManus et al., RNA 8:842-850 (2002); Sui et al., Proc. Natl. Acad. Sci. USA 99(6):5515-5520 (2002)).

The nucleic acid molecules or constructs can include dsRNA molecules comprising 16-30, e.g., 16, 17, 18, 19, 20, 21, 22, 23, 24, 25, 26, 27, 28, 29, or 30 nucleotides in each strand, wherein one of the strands is substantially identical, e.g., at least 80% (or more, e.g., 85%, 90%, 95%, or 100%) identical, e.g., having 3, 2, 1, or 0 mismatched nucleotide(s), to a target region in the mRNA, and the other strand is complementary to the first strand. The dsRNA molecules can be chemically synthesized, or can transcribed be *in vitro* from a DNA template, or *in vivo* from, e.g., shRNA. The dsRNA molecules can be designed using any method known in the art; a number of algorithms are known, and are commercially available. Gene walk methods can be used to optimize the inhibitory activity of the siRNA.

The nucleic acid compositions can include both siRNA and modified siRNA derivatives, e.g., siRNAs modified to alter a property such as the pharmacokinetics of the composition, for example, to increase half-life in the body, as well as engineered RNAi precursors.

siRNAs can be delivered into cells by methods known in the art, e.g., cationic liposome transfection and electroporation. siRNA duplexes can be expressed within cells from engineered RNAi precursors, e.g., recombinant DNA constructs using mammalian Pol III promoter systems (e.g., H1 or U6/snRNA promoter systems (Tuschl (2002), *supra*) capable of expressing functional double-stranded siRNAs; (Bagella et al., J. Cell. Physiol. 177:206-213 (1998); Lee *et al.* (2002), *supra*; Miyagishi *et al.* (2002), *supra*; Paul *et al.* (2002), *supra*; Yu *et al.* (2002), *supra*; Sui *et al.* (2002), *supra*). Transcriptional termination by RNA Pol III occurs at runs of four consecutive T residues in the DNA template, providing a mechanism to end the siRNA transcript at a specific sequence. The siRNA is complementary to the sequence of the target gene in 5'-3' and 3'-5' orientations, and the two strands of the siRNA can be expressed in the same construct or in separate constructs. Hairpin siRNAs, driven by H1 or U6 snRNA promoter and expressed in cells, can inhibit target gene expression (Bagella *et al.* (1998), *supra*; Lee *et al.* (2002), *supra*; Miyagishi *et al.* (2002), *sura;* Paul *et al.* (2002), *supra*; Yu *et al.* (2002), *supra*; Sui *et al.* (2002) *supra*). Constructs containing siRNA sequence under the control of T7 promoter also make functional siRNAs when cotransfected into the cells with a vector expression T7 RNA polymerase (Jacque (2002), *supra*).

### Antisense

An "antisense" nucleic acid can include a nucleotide sequence that is complementary to a "sense" nucleic acid encoding a protein, e.g., complementary to the coding strand of a double-stranded cDNA molecule or complementary to a PKCd mRNA sequence. The antisense nucleic acid can be complementary to an entire coding strand of a target sequence, or to only a portion thereof. In another embodiment, the antisense nucleic acid molecule is antisense to a "noncoding region" of the coding strand of a nucleotide sequence (e.g., the 5' and 3' untranslated regions).

An antisense nucleic acid can be designed such that it is complementary to the entire coding region of a target mRNA, but can also be an oligonucleotide that is antisense to only a portion of the coding or noncoding region of the target mRNA. For example, the antisense oligonucleotide can be complementary to the region surrounding the translation start site of the target mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

Based upon the sequences disclosed herein, one of skill in the art can easily choose and synthesize any of a number of appropriate antisense molecules for use in accordance with the present invention. For example, a "gene walk" comprising a series of oligonucleotides of 15-30 nucleotides spanning the length of a target nucleic acid can be prepared, followed by testing for inhibition of target gene expression. Optionally, gaps of 5-10 nucleotides can be left between the oligonucleotides to reduce the number of oligonucleotides synthesized and tested. Such methods can also be used to identify siRNAs.

In some embodiments, the antisense nucleic acid molecule is an α-anomeric nucleic acid molecule. An α-anomeric nucleic acid molecule forms specific double-stranded hybrids with complementary RNA in which, contrary to the usual β-units, the strands run parallel to each other (Gaultier et al., Nucleic Acids. Res. 15:6625-6641 (1987)). The antisense nucleic acid molecule can also comprise a 2'-o-methylribonucleotide (Inoue et al. Nucleic Acids Res. 15:6131-6148 (1987)) or a chimeric RNA-DNA analogue (Inoue et al. FEBS Lett., 215:327-330 (1987)).

In some embodiments, the antisense nucleic acid is a morpholino oligonucleotide (see, e.g., Heasman, Dev. Biol. 243:209-14 (2002); Iversen, Curr. Opin. Mol. Ther. 3:235-8 (2001); Summerton, Biochim. Biophys. Acta. 1489:141-58 (1999).

Target gene expression can be inhibited by targeting nucleotide sequences complementary to a regulatory region (e.g., promoters and/or enhancers) to form triple helical structures that prevent transcription of the Spt5 gene in target cells. See generally, Helene, C. Anticancer Drug Des. 6:569-84 (1991); Helene, C. Ann. N.Y. Acad. Sci. 660:27-36 (1992); and Maher, Bioassays 14:807-15 (1992). The potential sequences that can be targeted for triple helix formation can be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

Antisense molecules targeting PKCd are described in US6339066; US6235723; and WO0070091. All of the foregoing are incorporated by reference herein.

### Ribozymes

Ribozymes are a type of RNA that can be engineered to enzymatically cleave and inactivate other RNA targets in a specific, sequence-dependent fashion. By cleaving the target RNA, ribozymes inhibit translation, thus preventing the expression of the target gene. Ribozymes can be chemically synthesized in the laboratory and structurally modified to increase their stability and catalytic activity using methods known in the art. Alternatively, ribozyme genes can be introduced into cells through gene-delivery mechanisms known in the art. A ribozyme having specificity for a target nucleic acid can include one or more sequences complementary to the nucleotide sequence of a cDNA described herein, and a sequence having known catalytic sequence responsible for mRNA cleavage (see U.S. Pat. No. 5,093,246 or Haselhoff and Gerlach Nature 334:585-591 (1988)). For example, a derivative of a Tetrahymena L-19 IVS RNA can be constructed in which the nucleotide sequence of the active site is complementary to the nucleotide sequence to be cleaved in a target mRNA. See, e.g., Cech et al. U.S. Patent No. 4,987,071; and Cech et al. U.S. Patent No. 5,116,742. Alternatively, a target mRNA can be used to select a catalytic RNA having a specific ribonuclease activity from a pool of RNA molecules. See, e.g., Bartel and Szostak, Science 261:1411-1418 (1993).

### Aptamers

Aptamers are short oligonucleotide sequences which can specifically bind specific proteins. It has been demonstrated that different aptameric sequences can bind specifically to different proteins, for example, the sequence GGNNGG where N=guanosine (G), cytosine (C), adenosine (A) or thymidine (T) binds specifically to thrombin (Bock et al (1992) Nature 355: 564 566 and U.S. Pat. No. 5,582,981 (1996) Toole et al). Methods for selection and preparation of such RNA aptamers are knotn in the art (see, e.g., Famulok, Curr. Opin. Struct. Biol. 9:324 (1999); Herman and Patel, J. Science 287:820-825 (2000)); Kelly et al., J. Mol. Biol. 256:417 (1996); and Feigon et al., Chem. Biol. 3: 611 (1996)).

### Dosage

An "effective amount" is an amount sufficient to effect beneficial or desired results. For example, a therapeutic amount is one that achieves the desired therapeutic effect, as described herein. This amount can be the same or different from a prophylactically effective amount, which is an amount necessary to prevent onset of disease or disease symptoms. An effective amount can be administered in one or more administrations, applications or dosages. A therapeutically effective amount of a therapeutic compound (i.e., an effective dosage) depends on the therapeutic compounds selected. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the therapeutic compounds described herein can include a single treatment or a series of treatments.

Dosage, toxicity and therapeutic efficacy of the therapeutic compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### Pharmaceutical Conapositions and Methods of Administration

Pharmaceutical compositions typically include a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration.

Pharmaceutical compositions are typically formulated to be compatible with its intended route of administration. Examples of routes of administration include parenteral, e.g., intravenous, intradermal, subcutaneous, oral (e.g., inhalation), transdermal (topical), transmucosal, and rectal administration. In some embodiments, e.g., for the treatment of fatty liver disease, the methods include local administration to the liver.

Methods of formulating suitable pharmaceutical compositions are known in the art, see, e.g., the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressured container or dispenser that contains a suitable propellant, e.g., a gas such as carbon dioxide, or a nebulizer. Such methods include those described in U.S. Patent No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The pharmaceutical compositions can also be prepared in the form of suppositories (e.g., with conventional suppository bases such as cocoa butter and other glycerides) or retention enemas for rectal delivery.

The inhibitory nucleic acid molecules described herein can be administered to a subject (e.g., by direct injection at a tissue site), or generated *in situ* such that they hybridize with or bind to cellular mRNA and/or genomic DNA encoding a target protein to thereby inhibit expression of the protein, e.g., by inhibiting transcription and/or translation. Alternatively, inhibitory nucleic acid molecules can be modified to target selected cells and then administered systemically. For systemic administration, inhibitory nucleic acid molecules can be modified such that they specifically bind to receptors or antigens expressed on a selected cell surface, e.g., by linking the inhibitory nucleic acid nucleic acid molecules to peptides or antibodies that bind to cell surface receptors or antigens. The inhibitory nucleic acid nucleic acid molecules can also be delivered to cells using vectors known in the art of described herein. To achieve sufficient intracellular concentrations of the inhibitory nucleic acid molecules, vector constructs in which the inhibitory nucleic acid nucleic acid molecule is placed under the control of a strong promoter can be used.

Therapeutic compounds that are or include nucleic acids can be administered by any method suitable for administration of nucleic acid agents, such as a DNA vaccine. These methods include gene guns, bio injectors, and skin patches as well as needle-free methods such as the micro-particle DNA vaccine technology disclosed in U.S. Patent No. 6,194,389, and the mammalian transdermal needle-free vaccination with powder-form vaccine as disclosed in U.S. Patent No. 6,168,587. Additionally, intranasal delivery is possible, as described in, *inter alia,* Hamajima et al., Clin. Immunol. Immunopathol., 88(2), 205-10 (1998). Liposomes (e.g., as described in U.S. Patent No. 6,472,375) and microencapsulation can also be used. Biodegradable targetable microparticle delivery systems can also be used (e.g., as described in U.S. Patent No. 6,471,996).

In one embodiment, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or obtained commercially, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

The pharmaceutical compositions can be included in a container, pack, or dispenser together with instructions for administration.

### EXAMPLES

The invention is further described in the following examples, which do not limit the scope of the invention described in the claims.

### Mice

The following mice, diets and treatments were used in these Examples.

C57BL/6J male mice were obtained from Jackson Laboratory (Bar Harbor, ME), 129SvEv male mice were obtained from Taconic (Germantown, NY). Mice were maintained on a 12-hour (h) light-dark cycle with *ad libidum* access to tap water and standard chow diet containing 21% calories from fat, 22% protein, and 57% carbohydrates (PharmaServ, Framingham, MA). For some studies, 6 week-old mice were submitted to low-fat diet - 14% of calories from fat, 25% from protein, and 61 % from carbohydrates (Taconic) - or high-fat diet - 55% fat, 21% protein, and 24% carbohydrates (Harlan Teklad, Madison, WI) - for 18 weeks prior to the sacrifice. All protocols for animal use were reviewed and approved by the Animal Care Committee of the Joslin Diabetes Center and were in accordance with National Institutes of Health guidelines.

### Example 1. Increased Expression of PKCd in Liver is a Feature of Pro-Diabetic Mice

Compared to 129 mice, B6 mice develop more severe insulin resistance when subjected to genetic defects in insulin signaling or environmentally induced insulin resistance by high fat diet. Genome-wide association analysis reveals several regions which link to increased risk of insulin resistance in the B6 mouse, the strongest of which is D14Mit52, in the PKCd locus on chromosome 14 (Almind, K. and Kahn, C. R. (2004) Diabetes 53, 3274-3285). This non-coding SNP is associated with increased expression of PKCd in B6 mice.

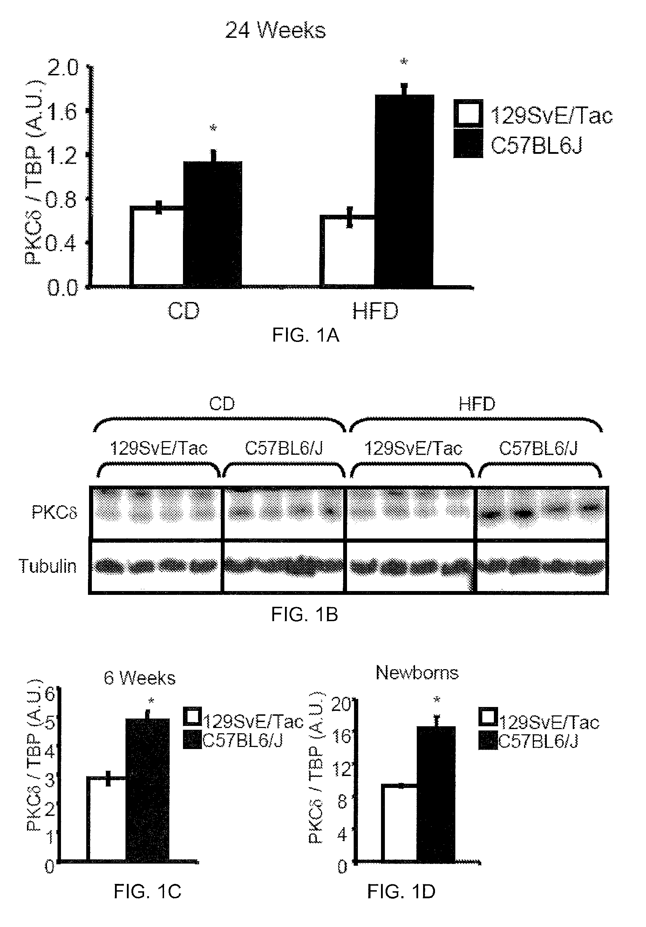

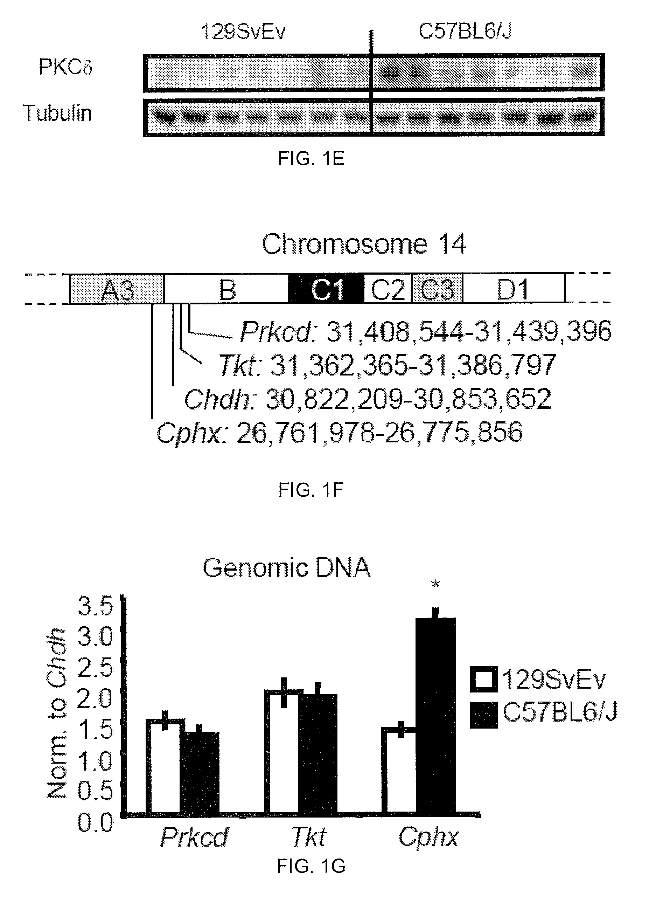

Expression of PKCd mRNA and protein was assayed in livers mice of various genotypes and under various conditions of diet. For all mRNA studies samples were prepared using RNeasy extraction kit according to the manufacturer protocol (QIAGEN), mRNA was measured by quantitative real-time PCR (qPRC), and results were normalized to TBP. For western blot analysis, 40-50 ug of each extract was loaded per lane. Each lane represents an individual animal. On a normal chow diet at 24 weeks of age, B6 mice have ∼ 60% higher levels of PKCd mRNA in liver than 129 mice (1.11 ± 0.11 vs. 0.71 ± 0.04, p<0.02) (Fig. 1A). Moreover, B6 and 129 mice were placed on high fat diet (HFD) for 18 weeks starting a 6 weeks of age, hepatic PKCd expression further increased by two-fold in B6 mice, but did not increase in 129 mice, leading to three-fold higher expression in the B6 vs. 129 mice (Fig. 1A). These differences in PKCd messenger expression lead to similar differences in expression at the protein level when assessed by Western blot analysis of liver extracts (Fig. 1B). This difference in PKCd expression between B6 and 129 mice was also observed at 6 weeks of age (Fig. 1C), when 129 mice actually weigh slightly more than the B6 (22.1 ± 0.5 vs. 19.8 ± 0.3 g, p<0.001) and have identical glucose and insulin levels. More importantly, the increased expression of PKCd in B6 vs. 129 mice was also true at both mRNA and protein levels for livers of newborn mice (Figs. 1D-E), suggesting a genetically driven differential expression for PKCd between insulin resistance-prone C57BL6/J and insulin resistance-resistant 129SvEv mice.

Recent studies have revealed that one cause for gene expression differences between mouse strains is duplication or deletion of segments of DNA resulting in variation in gene copy number. Copy number variations have been identified in chromosome 14 near the *Prkcd* locus between commonly used mice lines, including B6 and 129 (She, X., Cheng, Z., Zollner, S., Church, D. M., and Eichler, E. E. (2008) Nat.Genet. 40, 909-914). To explore whether this could account for the differences in expression of PKCd, we assessed the relative copy number of *Prkcd* gene and the nearby *Tkt* gene, as well as *Cphx* and *Chdh* genes located upstream and downstream of *Prkcd* on chromosome 14 using quantitative reverse transcription PCR analysis. Total RNA was isolated from mouse tissues using an RNeasy kit (QIAGEN, Valencia, California). cDNA was prepared from 1 µg of RNA using the Advantage RT-PCR kit (BD Biosciences, Palo Alto, California) with random hexamer primers, according to manufacturer's instructions. The resulting cDNA was diluted 10-fold, and a 5 µl aliquot was used in a 20 µl PCR reaction (SYBR Green, PE Biosystems) containing primers at a concentration of 300 nM each. PCR reactions were run in triplicate and quantitated in the ABI Prism 7700 Sequence Detection System. Ct values were normalized to level of ribosomal 18S RNA. See Fig. 1F.

Since the *Chdh* gene is known not to exhibit copy variations, the results for other genes could be normalized to *Chdh* to assess copy number variation. Consistent with previous reports indicating duplication in this region (She, X., Cheng, Z., Zollner, S., Church, D. M., and Eichler, E. E. (2008) Nat.Genet. 40, 909-914), we observed a 2-fold increase in copy number for the *Cphx* gene in B6 mice compared to 129 mice (p<0.0001). However, genomic PCR revealed no differences on chromosome 14 for *Prkcd* gene or the nearby *Tkt* gene (Fig. 1G).

### Example 2. Differential Regulation of Hepatic PKCd Expression Between B6 and 129 Mice

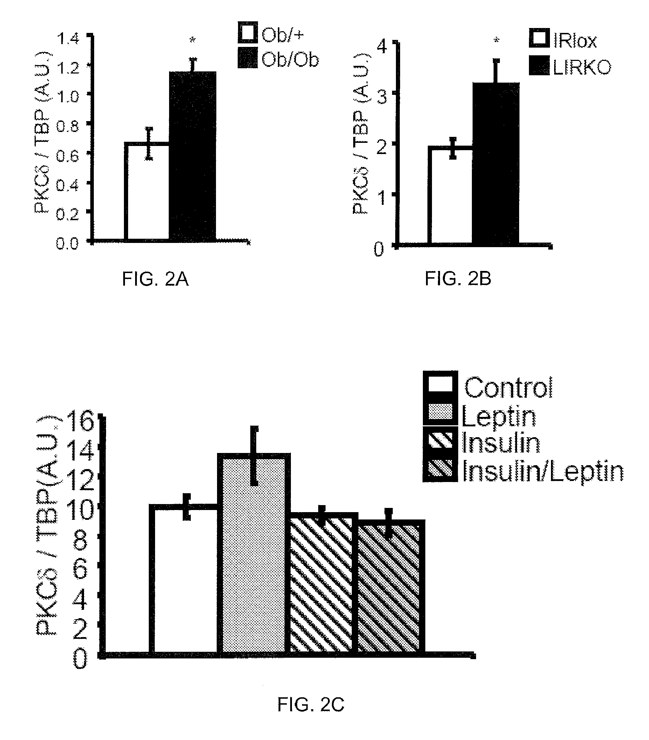

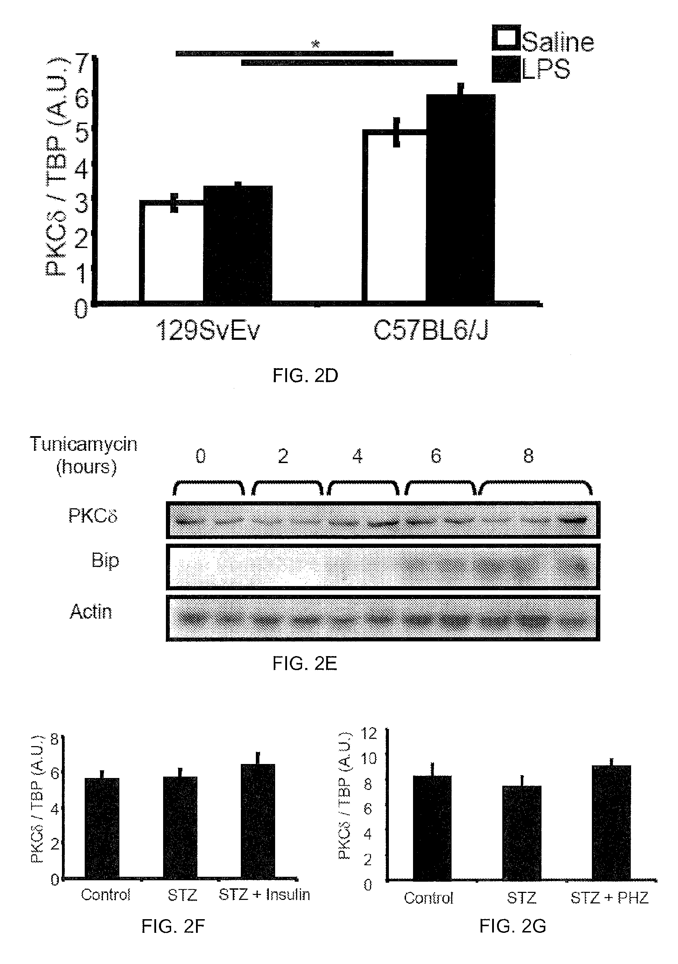

Although the difference in PKCd expression can be observed between B6 and 129 mice prior to the onset of obesity, obesity and insulin resistance also regulate PKCd. Thus, increased expression of PKCd in liver was also observed in ob/ob mice vs. control on a B6 background (11.35 ± 1.00 vs. 6.60 ± 1.05, p<0.005) (Fig. 2A) and this was not reversed by short-term infusion of insulin and/or leptin (Fig. 2C). Leptin treatment was performed as follows. Male ob/ob mice (aged 6 weeks) and their lean ob control littermates were purchased from Jackson Laboratories and acclimated to our animal facility for 2 weeks before study. Mice were implanted with osmotic pumps (1007D; Alzet, Cupertino, CA) containing either PBS or recombinant mouse leptin (24 µg/day; National Hormone and Peptide Program, Harbor-UCLA Medical Center, Torrance, CA) for 4 days. Insulin-treated mice also received four subcutaneous bovine insulin pellets (Linbit; Linshin, Toronto, ON, Canada), a dose found to raise insulin levels in leptin-treated ob/ob mice to those found in untreated ob/ob mice. Fasting insulin and glucose levels were obtained in a cohort of ob/ob mice by subjecting them to a 6-h fast 2 days after initiating treatment with leptin or insulin.

There was also an increase in PKCd in livers of liver-specific insulin receptor knock-out (LIRKO) vs. their lox/lox control on a mixed B6/129 background (3.16 ± 0.48 vs. 1.91 ± 0.19, p<0.05) (Fig. 2B).

Since obesity is associated with increased inflammation in fat and C57BL6/J and 129SvEv mice differ in the level of inflammation that occurs in response to HFD-induced obesity (Mori, M. A., Liu, M., Bezy, O., Almind, K., Shapiro, H., Kasif, S., and Kahn, C. R. (2010), "A Systems Biology Approach Identifies Inflammatory Abnormalities between Mouse Strains Prior to Development of Metabolic Disease" Diabetes [Epub; PMID: 20713682), we tested whether injection of bacterial lipopolysaccharide (LPS), which triggers a massive, generalized inflammatory response, would lead to changes the expression of PKCd.

To induce an inflammation in vivo, mice were injected intraperitoneally with 100 µg LPS (*Escherichia coli* 55:B5; Sigma) or sterile saline as control. Mice were sacrificed 18 hours post-injection. For ER stress studies, sibling mice (6- to 10-week-old) were given a single 1µg/g body weight intraperitoneal injection of a 0.05 mg/ml suspension of tunicamycin and sacrificed 2, 4, 6 and 8 hours post-injection.

While B6 mice had higher basal levels of PKCd expression, no significant induction of PKCd was observed in response to LPS treatment in either strain (Fig. 2D). Likewise, endoplasmic reticulum (ER) stress has been shown to be associated with insulin resistance (Ozcan, U., Cao, Q., Yilmaz, E., Lee, A. H., Iwakoshi, N. N., Ozdelen, E., Tuncman, G., Gorgun, C., Glimcher, L. H., and Hotamisligil, G. S. (2004) Science 306, 457-461), and several studies have suggested a possible association between ER stress and PKCd (Greene, M. W., Ruhoff, M. S., Burrington, C. M., Garofalo, R. S., and Orena, S. J. (2010) Cell Signal. 22, 274-284; Qi, X. and Mochly-Rosen, D. (2008) J Cell Sci 121, 804-813). To assess the possibility of PKCd induction being a consequence of ER stress, we studied PKCd protein expression in liver during following tunicamycin injection, a potent pharmaceutical inducer of ER stress. As shown in Figure 2E, we did not observe any alterations in PKCd expression during the onset of ER stress, whereas the ER stress chaperone protein Bip was clearly induced by tunicamycin treatment. Finally, to determine if hyperglycemia or insulin itself might directly regulate PKCd expression, we treated mice with streptozotocin to induce insulin-deficient diabetes and rescued some of the STZ-diabetic mice with insulin or phlorizin treatment to normalize their blood glucose levels.

Streptozotocin treatment was performed as follows. Mice at 6 weeks of age received either daily i.p. injections of sodium citrate (pH 4.3) for controls or STZ (Sigma) resuspended in sodium citrate, 100 µg per g of body weight for three consecutive days. When these mice achieved fed glucose levels of >400 mg/dl for three consecutive days, they were separated into two equal groups. One group was not treated, while the other were treated with s.c. insulin pellets (LinShin, Toronto, ON, Canada), to obtain fed glucose levels of <200 mg/dl for at least three consecutive days. For phlorizin treatment of diabetic mice, male 8-week-old C57B1/6 mice were treated with a single intraperitoneal injection (200 µg/g body weight) of STZ (Sigma). PHZ (Sigma) was dissolved in a solution containing 10% ethanol, 15% DMSO, and 75% saline and was injected subcutaneously at a dose of 0.4 g/kg twice daily for 10 days from 8 days after the STZ injection. Control mice were injected with the same volume of vehicle.

Again, no differences in PKCd expression were observed in either the STZ diabetic mice or the insulin- or phlorizin-treated groups (Fig. 2F-G), indicating that neither hyperglycemia, insulin, leptin, inflammation nor ER stress appear to be responsible for the high-fat diet or obesity-dependant induction of PKCd observed in the liver of B6 mice. Rather this seems to be an intrinsic genetically programmed difference between B6 and 129 mice strains.

### Example 3. PKCd knockout mice display improved glucose tolerance and insulin sensitivity.

To determine if these differences in PKCd expression might modulate insulin sensitivity, we studied three different mice models with different alterations in PKCd expression. These included: 1) mice with global inactivation of the *Prkcd* gene (PKCdKO) created by gene targeting (Leitges, M., Mayr, M., Braun, U., Mayr, U., Li, C., Pfister, G., Ghaffari-Tabrizi, N., Baier, G., Hu, Y., and Xu, Q. (2001) J Clin Invest 108, 1505-1512), mice with liver specific inactivation of the *Prkcd* gene created using the Cre-lox system of conditional recombination, and 3) mice with over-expression of PKCd in the liver created using adenoviral mediated gene transfer. Adenoviruses encoding PKCd or GFP were prepared as previously described (Taniguchi, C. M., Aleman, J. O., Ueki, K., Luo, J., Asano, T., Kaneto, H., Stephanopoulos, G., Cantley, L. C., and Kahn, C. R. (2007) Mol Cell Biol 27, 2830-2840) and injected into the tail vein 5 to 10 days prior to sacrifice. Adenoviruses encoding Cre Recombinase were purchased from Transfer Vector Core, University of Iowa (Iowa City, IA).

Metabolic studies were performed as follows. For glucose tolerance testing (GTT), blood samples were obtained at 0, 15, 30, 60, and 120 min after intraperitoneal injection of 2 g/kg dextrose. Insulin tolerance tests were performed by injecting 1 U/kg insulin (Novolin, Novo Nordisk, Denmark) intraperitoneally, followed by blood collection at 0, 15, 30, and 60 min. The pyruvate challenge was performed by injected 2 g/kg of pyruvate (Sigma) intraperitoneally, with blood glucose measured at 0, 15, 30, and 60 min time points. Blood glucose values were determined using a One Touch II glucose monitor (Lifescan Inc., Milipitas, California).

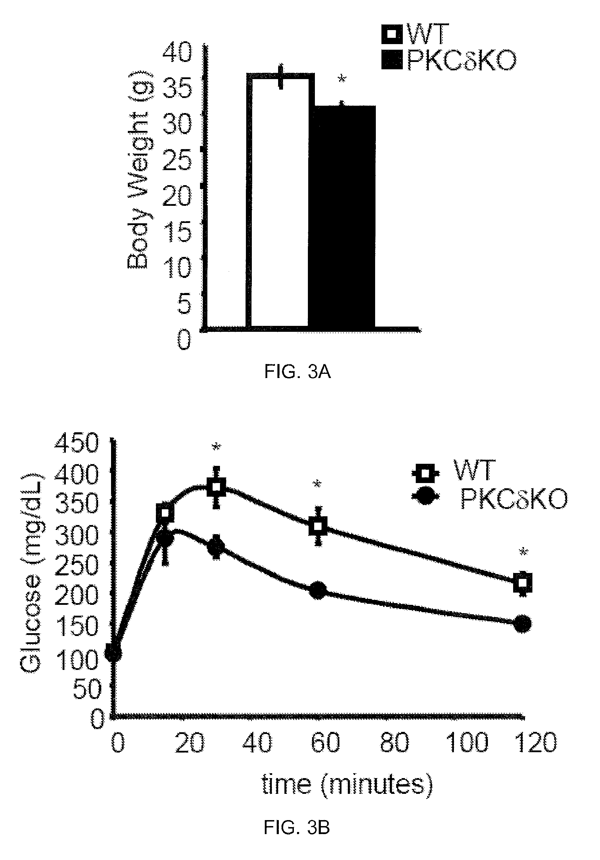

Mice with global inactivation of PKCd have been previously shown to have defects in immune system function (Miyamoto A. and al, Nature, 25 April 2002, vol.416, p.865). If surviving mice were fed a regular chow diet and analyzed at 20 weeks of age, the PKCdKO mice had significantly lower body weights than their wild-type mice (WT) littermates (30 ± 0.7 and 35 ± 1.3 g, p<0.05) (Fig. 3A). Intraperitoneal glucose tolerance testing (GTT) revealed that PKCdKO mice had significantly better glucose tolerance than their control littermates (Fig. 3B) with a 35% decrease of the AUC for glucose levels following the glucose challenge.

Hyperinsulinemic-euglycemic clamp experiments were performed as follows. The jugular vein of mice was cannulated, and after a 1 week of recovery period and a 4 hr fast, a hyperinsulinemic-euglycemic clamp was performed with D-[3-³H]glucose as previously described (Norris, A. W., Chen, L., Fisher, S. J., Szanto, I., Ristow, M., Jozsi, A. C., Hirshman, M. F., Rosen, E. D., Goodyear, L. J., Gonzalez, F. J., Spiegelman, B. M., and Kahn, C. R. (2003) J Clin Invest 112, 608-618), using a continuous insulin infusion of 3.5 mU/kg/min. Hepatic glucose production was assessed by subtraction of the glucose infusion rate from whole-body glucose turnover as measured with D-[3-³H]glucose.

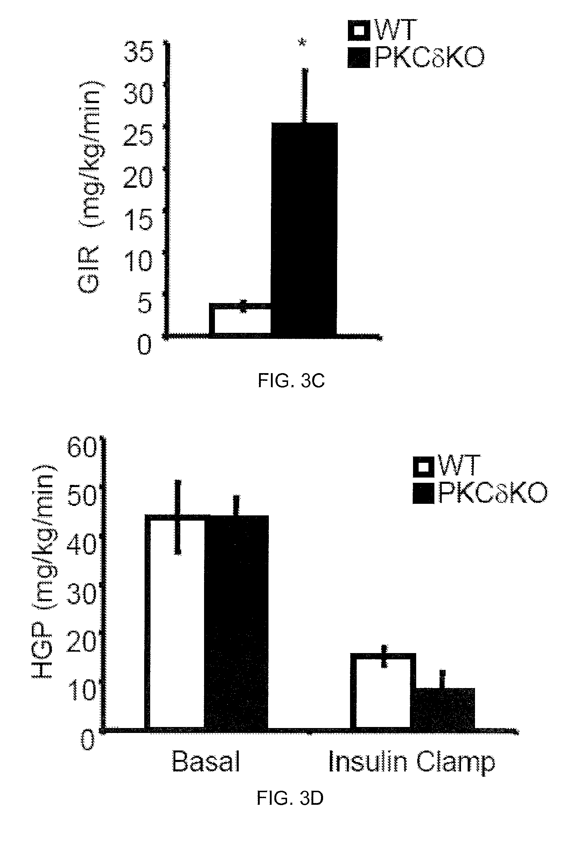

These hyperinsulinemic-euglycemic clamp studies confirmed the increased whole body insulin sensitivity with a striking and significant seven-fold increase in the glucose infusion rates in PKCd knockout mice compared to the WT mice (25.1 ± 6.5 vs. 3.5 ± 0.5 mg/kg/min, p<0.05) (Fig. 3C). This increase in insulin sensitivity in PKCdKO mice was accompanied by increased insulin sensitivity of the liver with an increased ability of insulin to repress hepatic glucose production (HPG). In the basal state, WT and PKCdKO mice had similar levels of HGP, but during the insulin clamp, PKCdKO had significantly lower HPG than the WT mice. Therefore, PKCdKO mice displayed an 83% ± 6.9 inhibition of HPG versus only 63% ± 8.9 inhibition for the WT mice (Fig. 3D), indicating enhanced ability of insulin to suspress gluconeo genesis.

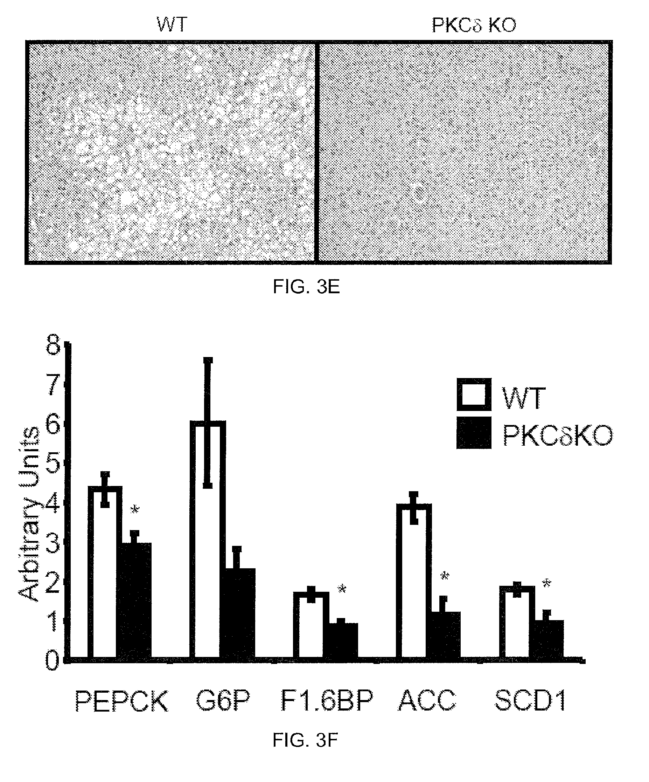

This difference in ability of insulin to suppress gluconeogenesis was due to differences in insulin-regulated gene expression. Thus, the PKCdKO mice had significant lower mRNA levels of the key enzymes of gluconeogenic pathway, including a 25% decrease in phosphoenolpyruvate carboxykinase (PEPCK), a 60% decrease in glucose-6-phospatase (G6P) and a 55% decrease fructose-1,6-bisphosphatase (F1,6BP) (Fig. 3F). PKCd null mice also had decreased expression of the lipogenic enzymes acetyl-coA-carboxylase (ACC) (70%) and stearoyl-CoA desaturase 1 (SCD1) (55%). This reduction in expression of enzymes of the lipogenic pathway was accompanied by a protection against aging-related hepatosteatosis as shown in histological analysis of liver section from 24 months old PKCdKO mice (Fig. 3E).

PKCd ablation also improved hepatic insulin signaling as assessed by western blot analysis of liver extracts from mice taken during the hyperinsulinemic-euglycemic clamp as follows.

Following an overnight fast, mice were anesthetized with 2,2,2-tribromoethanol in PBS (Avertin), and injected with 5 U of regular human insulin (Novolin, Novo Nordisk, Denmark) via the inferior vena cava. Five minutes after the insulin bolus, tissues were removed and frozen in liquid nitrogen. Immunoprecipitation and immunoblot analysis of insulin signaling molecules were performed using tissue homogenates prepared in a tissue homogenization buffer that contained 25 mM Tris-HCl (pH 7.4), 10 mM Na₃VO₄, 100 mM NaF, 50 mM Na₄P₂O₇, 10 mM EGTA, 10 mM EDTA, 2 mM phenylmethylsulfonyl fluoride, 1% Nonidet-P40 supplemented with protease inhibitor cocktail (Sigma Aldrich). All protein expression data were quantified by densitometry using NIH Image. Rabbit polyclonal anti-Akt, anti-phospho Akt (Ser473), anti-phospho GSK3β (Ser9), anti-FoxO1, anti-GSK3, anti-phospho p70S6K (Thr389), anti-phospho p70S6K (Thr421/Ser424), anti-p70S6K, anti-lamin A/C and anti-tubulin were purchased from Cell Signaling Technology (Beverly, Massachusetts). Anti-PKCd, anti-SREBP1c and anti-IRS1 were purchased from Santa Cruz. Anti-SOD4 was purchased from Abcam. Anti-phospho IRS1 (Ser307) was purchased from Upstate.

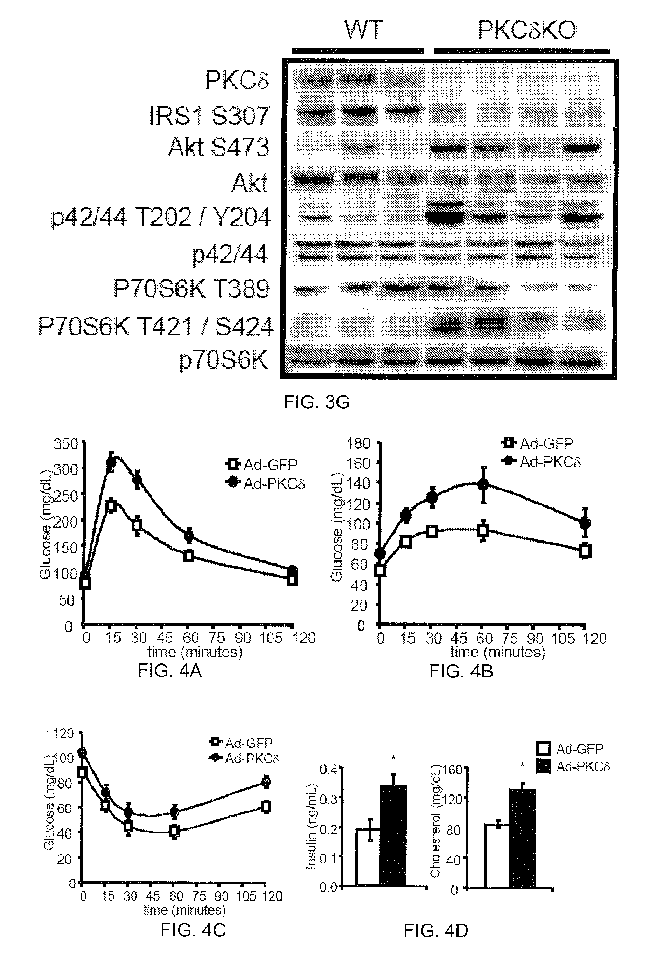

As shown in Figure 3G, PKCdKO mice had increased insulin stimulation of Akt phosphorylation and phosphorylation of the p42 and p44 MAP kinases. PKCdKO mice also had increased insulin-stimulated phosphorylation of p70S6Kinase on Thr421/Ser424, a site known to inhibit p70S6K activity (Dennis, P. B., Pullen, N., Pearson, R. B., Kozma, S. C., and Thomas, G. (1998) J Biol Chem. 273, 14845-14852; Um, S. H., Frigerio, F., Watanabe, M., Picard, F., Joaquin, M., Sticker, M., Fumagalli, S., Allegrini, P. R., Kozma, S. C., Auwerx, J., and Thomas, G. (2004) Nature. 431, 200-205). As a consequence, there was a decrease in the phosphorylation of IRS1 on Ser307, a known site for p70S6K phosphorylation (Um, S. H., Frigerio, F., Watanabe, M., Picard, F., Joaquin, M., Sticker, M., Fumagalli, S., Allegrini, P. R., Kozma, S. C., Auwerx, J., and Thomas, G. (2004) Nature. 431, 200-205), which was almost undetectable in PKCdKO mice. Taken together, these results demonstrate that whole body deletion of PKCd improves general insulin sensitivity and improves insulin action especially at the level of the liver.

### Example 4. Liver-specific overexpression of PKCd leads to metabolic syndrome symptoms.

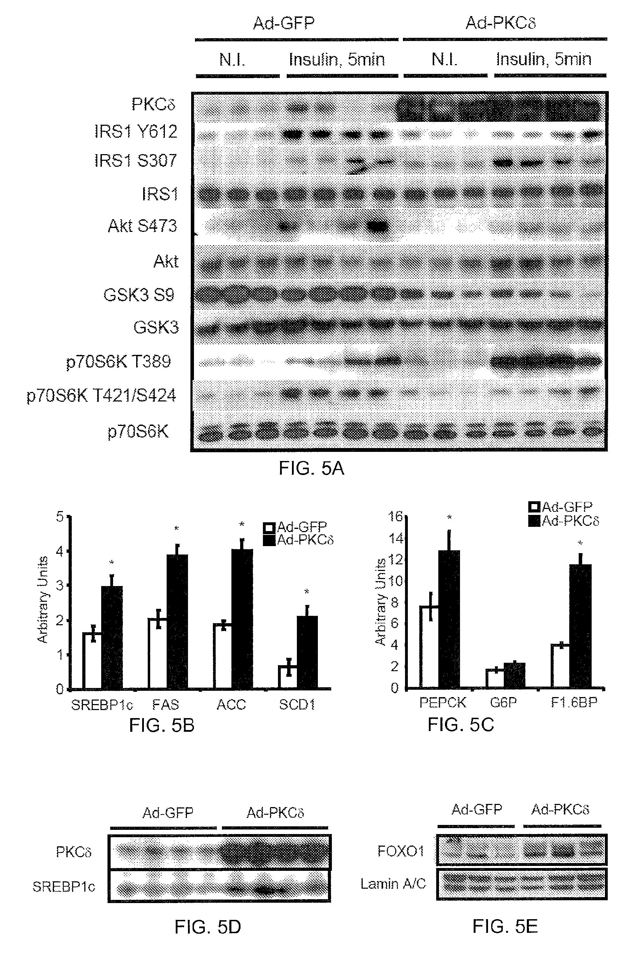

The data above indicate that high expression of PKCd in the liver is linked to hepatic insulin resistance. To directly define the role of increased PKCd in the liver, we created mice in which we had induced overexpression of PKCd in liver by adenoviral delivery of PKCd cDNA. This resulted in an eight-fold increase in the levels of PKCd in liver as determined by Western blot analysis (Fig. 5A).

On intraperitoneal glucose challenge (performed as described above), mice overexpressing PKCd were glucose intolerant in comparison with GFP-overexpressing control mice created in parallel (Fig. 4A). This resulted in a 30% increase in area under curve (AUC) of the glucose tolerance test (GTT). In distinction with the decreased gluconeogenesis in PKCd knockout, mice with increased levels of hepatic PKCd had increased gluconeogenesis as demonstrated by their exaggerated response to pyruvate tolerance test (PTT) (Figure 4B), with a 40% increase in AUC compared to control mice. This also resulted in a ∼20% increase in plasma glucose in both the fasted and fed states (cf. 0 time point in Figs. 4A, B and C, respectively). Although the basal glucose levels were increased, there was no difference the in the insulin-mediated reduction of glucose levels during an i.p. insulin-tolerance testing (ITT), suggesting that there was no difference in insulin sensitivity in muscle of between the mice overexpressing PKCd in liver and the controls (Fig 4C).

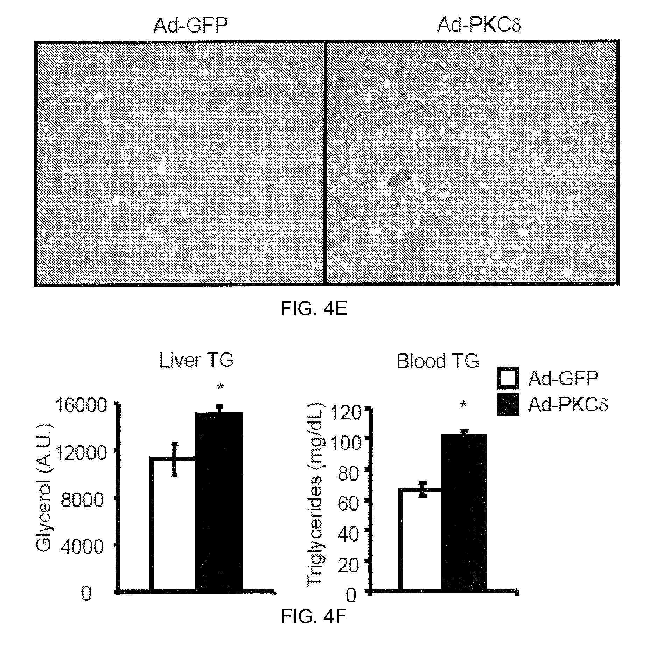

In addition to its effect on glucose metabolism, overexpression of PKCd in liver resulted in significant hepatosteatosis, which was easily observed in histological sections of liver stained with eosin (Fig. 4E). The increase in hepatic steatosis was further confirmed by measurements of triglyceride content of liver extracts which revealed a 33% increase in liver TG in mice overexpressing PKCd (Fig. 4F). Serum analysis also revealed that overexpression of PKCd in the liver created hyperglycemia (85 ± 3.6 vs. 69 ± 3.4 mg/dL in controls), hyperinsulinemia (0.33 ± 0.04 vs. 0.18 ± 0.03 ng/mL) and hypertriglyceridemia (101 ± 3.7 vs. 66 ± 4.0 mg/dL) (Fig 4D-F). Thus, overexpression of PKCd in liver reproduced many of the differences between 129 and B6 mice and the signs of metabolic syndrome, even in lean mice on regular chow diet.

### Example 5. PKCd overexpression alters hepatic insulin signaling.

The physiological alterations induced by PKCd overexpression were associated with hepatic insulin resistance at the level of the insulin signaling pathway. Thus, overexpression of PKCd decreased insulin-stimulated phosphorylation of IRS1 on Y612 and resulted in decreased phosphorylation of Akt and GSK3 on Ser473 and Ser9, respectively. As opposed to what had been observed in the PKCdKO mice, p70S6K phosphorylation on Thr389 in response to insulin was greatly enhanced by PKCd overexpression, and this correlated with the increased phosphorylation of IRS 1 on S307, a known p70S6K target site (Figure 5A). As a consequence of the repression of the insulin signaling, FOXO1 nuclear localization was enhanced about 2-fold in livers of mice overexpressing PKCd, as demonstrated by western blotting of nuclear extracts (Fig. 5E). Consistent with the role of FOXO1 as a regulator of hepatic gluconeogenesis (Puigserver, P., Rhee, J., Donovan, J., Walkey, C. J., Yoon, J. C., Oriente, F., Kitamura, Y., Altomonte, J., Dong, H., Accili, D., and Spiegelman, B. M. (2003) Nature. 423, 550-5550), mice overexpressing PKCd had from 1.2- to 2.5-fold increases in the levels of mRNA for the gluconeogenic enzymes PEPCK, G6P and F1,6BP (Fig. 5C).

Overexpression of PKCd also increased the levels of SREBP1c, a key hepatic transcription factor regulating lipogenesis, by 60% at both messenger and protein levels (Fig. 5B, C). This was accompanied by a two-fold increase in the expression of the two key lipogenic enzymes fatty acid synthase (FAS) and acetyl-coA carboxylase (ACC), both transcriptional targets of SREBP1c (Figure 5B). Thus, increasing the expression of PKCd by only a few fold is sufficient to create liver insulin resistance and to stimulate hepatic lipogenesis.

### Example 6. Liver-specific reduction of PKCd restores glucose tolerance and insulin signaling in high fat diet-induced diabetic animals.

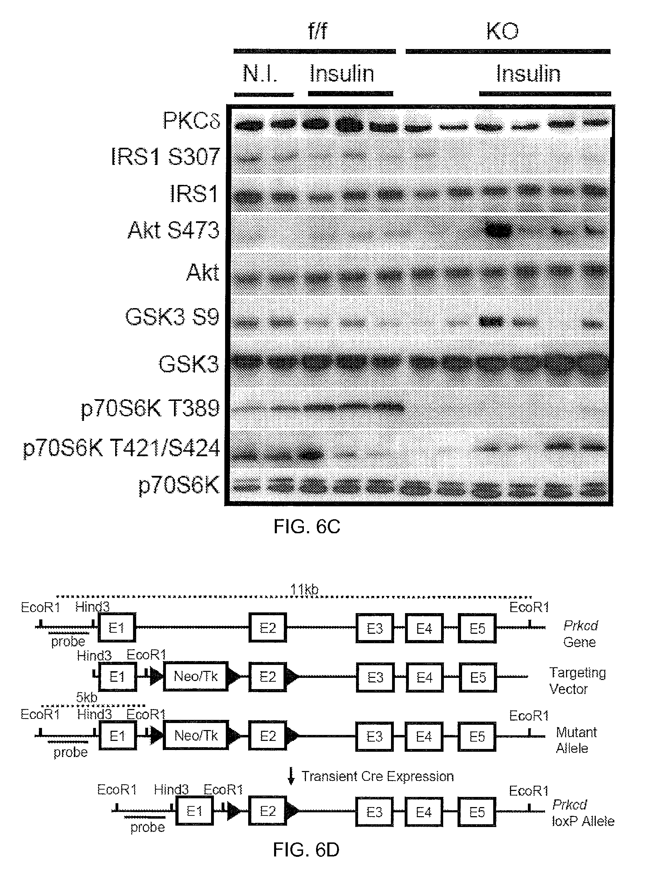

To determine if decreasing PKCd specifically in the liver could improve the hepatic insulin sensitivity in obese diabetic mice, we created a liver-specific PKCd knockout mouse. To this end, exon 2 of the *Prkcd* gene was flanked with two loxP sites and introduced in mice by homologous recombination (Fig. 6D). Loss of exon 2 of the floxed *Prkcd* gene would result in a deletion and frame-shift in the PKCd transcript, leading to the production of a truncated inactive PKCd protein. The constructs and mice were generated as follows.

Lambda phage clones containing the murine *Prkcd* gene were isolated from a 129 mouse ES cell library, and the targeting vector containing ∼ 9 kb in the two homology regions (Fig. 6D) was created and electroporated into E14/1 mouse ES cells. Stably transfected cells were isolated by selection with G418 (350 µg/ml; Gibco), and clones were screened for the desired homologous recombination event. A 1.8-kb EcoRI/HindIII fragment was used as an external probe for Southern blot analysis of DNA digested with EcoRI.

ES cell clones were injected into C57BL/6J mouse blastocysts, which were then transferred to pseudopregnant C57B1/6J female mice. Chimeric animals were screened for presence of the recombinant floxed allele by PCR using primers flanking the first and third loxP sites. Chimeric males were mated to C57BL/6J females that carried a Cre transgene under the control of the adenovirus EIIa promoter (Jackson Laboratories), which targets expression of Cre recombinase to the early mouse embryo prior to implantation in the uterine wall. A mosaic pattern of expression is commonly observed with Cre-mediated recombination occurring in a wide range of tissues, including the germ cells that transmit the genetic alteration to progeny. Progeny were screened by PCR to select *Prkcd* loxP/+ offspring containing two loxP site surrounding exon 2 and no Neo cassette. Offspring were analyzed by PCR of tail DNA to identify the *Prkcd* loxP/+ heterozygote mice. Tail DNA was extracted with DNeasy blood & tissue kit from QIAGEN (Valencia, CA). PKCdKO mice were genotyped with the following primers:
PKCD-Fwd: 5' - gCT CTA TTg CCT Cgg CTT CAT- 3' (SEQ ID NO:1);
PKCD-R: 5' - Agg TgA gAA gAC AgC AAA ggg - 3' (SEQ ID NO:2);
LacZ-F: 5' - TgA TgC ggT gCT gAT TAC gAC - 3' (SEQ ID NO:3);
LacZ-R: 5' - gTC AAA ACA ggC ggC AgT AAg - 3' (SEQ ID NO:4).
PKCdflox animals were genotyped with the following primers:
Primer F (Lox F3): 5' - CTg CTg ggT AAC TTA ACA ACA AgA CC - 3 '(SEQ ID NO:5); Primer R (Lox R (101)): 5' -CTg CTA AAT AAC ATg ATg TTC ggT CC-3' (SEQ ID NO:6); and Prkcdflox recomb 1744: 5' -gTA ggg TTg gAA ggg TCC CTA gA - 3' (SEQ ID NO:7).

Heterozygotes were mated to generate homozygous *Prkcd* loxP/loxP mice. The *Prkcd* loxP/loxP mice were backcrossed for 14 generations with C57BL/6J to obtain a homogeneous B6 background.

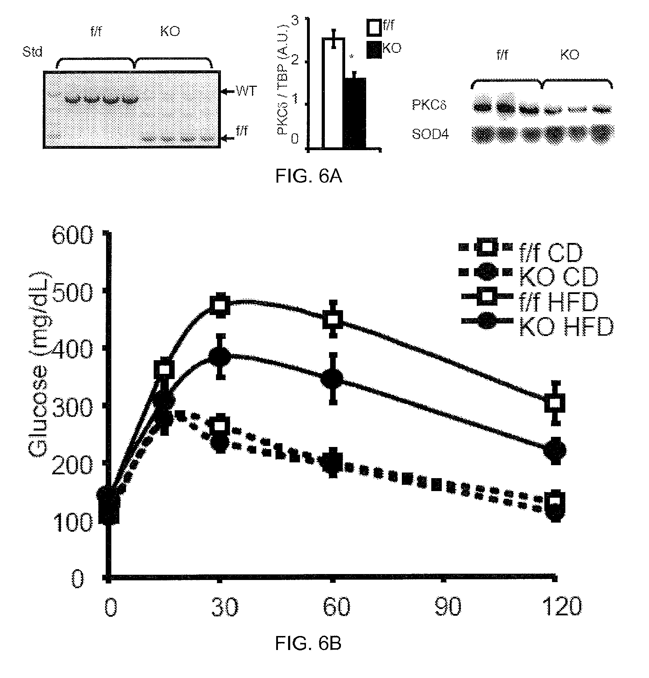

Intravenous administration of adenoviral vector containing Cre Recombinase expression cassette resulted in recombination of the PKCd transgene in liver of injected mice. At the dose of adenovirus used, five days after injection, there was a reproducible 40% reduction in PKCd mRNA and protein compared to control mice injected with empty adenoviral vector (Fig. 6A).

To assess the consequences of reduced levels of PKCd on hepatic insulin resistance, we injected empty or Cre Recombinase-expressing adenoviruses in 3 month old PKCd-floxed mice that had previously been subjected to 10 weeks of either a 55% high-fat diet or a regular 14% fat chow diet. Five days post adenovirus injection, there were no differences in body weight between the floxed mice and the mice with liver specific knockdown of PKCd on either the CD or HFD (data not shown). As expected, glucose tolerance testing demonstrated that mice that had been on high fat diet for 10 weeks were glucose intolerant compared to the chow diet fed control animals. In the lean animals, the modest reduction of PKCd had no effects on glucose tolerance. By contrast, in the high fat diet obese mice liver-specific reduction of PKCd was sufficient to significantly improve the glucose tolerance as compared to the obese controls (Fig. 6B) as demonstrated by a significant 30% reduction of the AUC for glucose.

This improvement in metabolism was secondary to an improvement in insulin signaling. Thus, in the obese HFD control mice, insulin signaling was markedly blunted with no detectable increase in Akt phosphorylation following insulin stimulation. This was due to high levels of inhibitory phosphorylation of IRS1 on Ser307 which appear to be secondary to high levels of p70S6K activation as indicated by p70S6 phosphorylation. Reducing PKCd reversed many of these changes. Thus, there was a reversal of the p70S6K phosphorylation/activation, leading to a reduction in IRS-1 Ser307 phosphorylation levels and a rescue of Akt/GSK3 phosphorylation/activation by insulin (Fig. 6C).

Despite the improvement in glucose tolerance, this short-term reduction of PKCd did not improve the hepatosteatosis present in mice after 10 weeks on high-fat diet as assessed by histological examination of liver sections. This result was confirmed by measurement of liver triglycerides content, which showed no reduction in the PKCd liver knockdown mice. Likewise, there was no change in the level of hyperinsulinemia, hypertriglyceridemia or free fatty acids induced by high fat diet. Therefore, a short-term reduction of hepatic PKCd was enough to rescue glucose tolerance and insulin signaling in the liver of high-fat diet fed mice, but not sufficient to reverse the hyperinsulinemia secondary or the lipid abnormalities in these mice.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Further aspects of the invention are disclosed in the following numbered clauses:
1. Use of a specific inhibitor of Protein Kinase C delta isoform (PKCd) for improving insulin sensitivity in a mammal.
2. Use of a specific inhibitor of Protein Kinase C delta isoform (PKCd) for treating or preventing fatty liver disease (FLD) in a mammal.
3. The use of clauses 1 or 3, wherein the mammal is obese or has visceral adiposity.
4. The use of clauses 1 or 3, wherein the mammal has type 2 diabetes.
5. The use of clause 3, wherein the mammal has Nonalcoholic Steatohepatitis (NASH) or is at risk of developing NASH.
6. The use of any of the foregoing clauses, wherein the inhibitor is a small molecule inhibitor or a peptide inhibitor or peptidomimetic thereof.
7. The use of clause 6, wherein the inhibitor is selected from the group consisting of KAI-9803, rottlerin, bisindolylmaleimide I, bisindolylmaleimide II, bisindolylmaleimide III, bisindolylmaleimide IV, calphostin C, chelerythrine chloride, ellagic acid, Go 7874, Go 6983, H-7, Iso-H-7, hypericin, K-252a, K- 252b, K-252c, melittin, NGIC-I, phloretin, staurosporine, polymyxin B sulfate, protein kinase C inhibitor peptide 19-31, protein kinase C inhibitor peptide 19-36, protein kinase C inhibitor (EGF-R Fragment 651-658, myristoylated), KID-1, Ro- 31-8220, Ro-32-0432, safmgol, sangivamycin, and D-erythro-sphingosine.
8. The use of any of clauses 1-5, wherein the inhibitor is a PKC delta-specific inhibitory nucleic acid.
9. The use of clause 8, wherein the PKC delta- specific inhibitory nucleic acid is a small interfering RNA or antisense specifically targeting PKC delta.
10. A method of improving insulin sensitivity in a mammal, the method comprising selecting a mammal on the basis that they are in need of improved insulin sensitivity; and administering to the mammal a therapeutically effective dose of one or more specific inhibitors of Protein Kinase C delta isoform (PKCd).
11. The method of clause 10, further comprising evaluating insulin sensitivity in the subject, before, during, or after administration of the inhibitor.
12. A method of treating or preventing fatty liver disease (FLD) in a mammal, the method comprising selecting mammal on the basis that they have or are at risk of developing FLD, and administering to the mammal a therapeutically effective dose of one or more specific inhibitors of Protein Kinase C delta isoform (PKCd).
13. The method of clause 12, further comprising evaluating fatty liver disease in the subject, before, during, or after administration of the inhibitor.
14. The method of clauses 10 or 12, wherein the mammal is obese or has visceral adiposity.
15. The method of clauses 10 or 12, wherein the mammal has type 2 diabetes.
16. The method of clause 12, wherein the mammal has Nonalcoholic Steatohepatitis (NASH) or is at risk of developing NASH.
17. The method of any of clauses 10-16, wherein the inhibitor is a small molecule inhibitor or a peptide inhibitor or peptidomimetic thereof.
18. The method of clause 17, wherein the inhibitor is selected from the group
   consisting of KAI-9803, rottlerin, bisindolylmaleimide I, bisindolylmaleimide II, bisindolylmaleimide III, bisindolylmaleimide IV, calphostin C, chelerythrine chloride, ellagic acid, Go 7874, Go 6983, H-7, Iso-H-7, hypericin, K-252a, K- 252b, K-252c, melittin, NGIC-I, phloretin, staurosporine, polymyxin B sulfate, protein kinase C inhibitor peptide 19-31, protein kinase C inhibitor peptide 19-36, protein kinase C inhibitor (EGF-R Fragment 651-658, myristoylated), KID-1, Ro- 31-8220, Ro-32-0432, safmgol, sangivamycin, and D-erythro-sphingosine.
19. The method of any of clauses 10-16, wherein the inhibitor is a PKC delta-specific inhibitory nucleic acid.
20. The method of clause 19, wherein the PKC delta-specific inhibitory nucleic acid is a small interfering RNA or antisense.

## Claims

1. Use of a specific inhibitor of Protein Kinase C delta isoform (PKCd) for improving insulin sensitivity in a mammal, wherein the inhibitor is KAI-9803.

2. Use of a specific inhibitor of Protein Kinase C delta isoform (PKCd) for treating or preventing fatty liver disease (FLD) in a mammal, wherein the inhibitor is KAI-9803.

3. The use of claims 1 or 2, wherein the mammal is obese or has visceral adiposity.

4. The use of claims 1 or 2, wherein the mammal has type 2 diabetes.

5. The use of claim 3, wherein the mammal has Nonalcoholic Steatohepatitis (NASH) or is at risk of developing NASH.

6. A specific inhibitor of Protein Kinase C delta isoform (PKCd) for use in a method of improving insulin sensitivity in a mammal, the method comprising selecting a mammal on the basis that they are in need of improved insulin sensitivity; and administering to the mammal a therapeutically effective dose of one or more specific inhibitors of Protein Kinase C delta isoform (PKCd), wherein the inhibitor is KAI-9803.

7. The inhibitor of claim 6, the method further comprising evaluating insulin sensitivity in the subject, before, during, or after administration of the inhibitor.

8. A specific inhibitor of Protein Kinase C delta isoform (PKCd) for use in a method of treating or preventing fatty liver disease (FLD) in a mammal, the method comprising selecting mammal on the basis that they have or are at risk of developing FLD, and administering to the mammal a therapeutically effective dose of one or more specific inhibitors of Protein Kinase C delta isoform (PKCd), wherein the inhibitor is KAI-9803.

9. The inhibitor of claim 8, further comprising evaluating fatty liver disease in the subject, before, during, or after administration of the inhibitor.

10. The inhibitor of claims 6 or 8, wherein the mammal is obese or has visceral adiposity; or wherein the mammal has type 2 diabetes.

11. The inhibitor of claim 8, wherein the mammal has Nonalcoholic Steatohepatitis (NASH) or is at risk of developing NASH.


SEQUENCE LISTING

<110> Joslin Diabetes Center, Inc.

<120> USE OF PROTEIN KINASE C DELTA (PKCD)
  INHIBITORS TO TREAT DIABETES, OBESITY, AND HEPATIC STEATOSIS

<130> PC926456EPA

<150> US 61/246,821
<151> 2009-09-29

<160> 7

<170> FastSEQ for Windows Version 4.0

<210> 1
<211> 21
<212> DNA
<213> Artificial Sequence

<220>
<223> synthetically generated oligonucleotide

<400> 1
gctctattgc ctcggcttca t                                                21

<210> 2
<211> 21
<212> DNA
<213> Artificial Sequence

<220>
<223> synthetically generated oligonucleotide

<400> 2
aggtgagaag acagcaaagg g                                                21

<210> 3
<211> 21
<212> DNA
<213> Artificial Sequence

<220>
<223> synthetically generated oligonucleotide

<400> 3
tgatgcggtg ctgattacga c                                                21

<210> 4
<211> 21
<212> DNA
<213> Artificial Sequence

<220>
<223> synthetically generated oligonucleotide

<400> 4
gtcaaaacag gcggcagtaa g                                                21

<210> 5

<211> 26
<212> DNA
<213> Artificial Sequence

<220>
<223> synthetically generated oligonucleotide

<400> 5
ctgctgggta acttaacaac aagacc                                            26

<210> 6
<211> 26
<212> DNA
<213> Artificial Sequence

<220>
<223> synthetically generated oligonucleotide

<400> 6
ctgctaaata acatgatgtt cggtcc                                            26

<210> 7
<211> 23
<212> DNA
<213> Artificial Sequence

<220>
<223> synthetically generated oligonucleotide

<400> 7
gtagggttgg aagggtccct aga                                               23